(19) Europäisches Patentamt
European Patent Office
Office européen des brevets

(11) Publication number: 0 391 665 A1

# (12) EUROPEAN PATENT APPLICATION

(21) Application number: 90303560.8

(22) Date of filing: 03.04.90

(51) Int. Cl.5: C07D 251/48, C08G 18/16, C08G 18/20, C08G 18/02

(30) Priority: 04.04.89 JP 85140/89
03.08.89 JP 202196/89

(43) Date of publication of application:
10.10.90 Bulletin 90/41

(84) Designated Contracting States:
DE FR GB NL

(71) Applicant: Hitachi Chemical Co., Ltd.
1-1, Nishishinjuku 2-chome
Shinjuku-ku, Tokyo 160(JP)

(72) Inventor: Semba, Reiko
410-1-19-503, Shimoozuki
Hadano-shi(JP)
Inventor: Hasebe, Mariko
5-18-204, Nakanarusawacho-2-chome
Hitachi-shi(JP)
Inventor: Moribe, Isamu
4-3, Suwacho-6-chome
Hitachi-shi(JP)
Inventor: Narushima, Ryoichi, San Haitsu
Taga 512
5-17, Higashikanesawacho-3-chome
Hitachi-shi(JP)
Inventor: Kikuchi, Tohru
5-D304, Nakanarusawacho-1-chome
Hitachi-shi(JP)
Inventor: Saito, Takayuki
3-6, Motomiyacho-4-chome
Hitachi-shi(JP)
Inventor: Kobayashi, Akihiro
Hitachi Kasei Shataku 234
4-6, Yushudainishi-1-chome, Ichihara-shi(JP)

(74) Representative: Cresswell, Thomas Anthony
et al
J.A. Kemp & Co. 14 South Square Gray's Inn
London WC1R 5LX(GB)

## (54) Amino compounds, amino resins obtained therefrom, production of amino resins and resin compositions for water-borne coatings.

(57) An amino compound of formula (I):

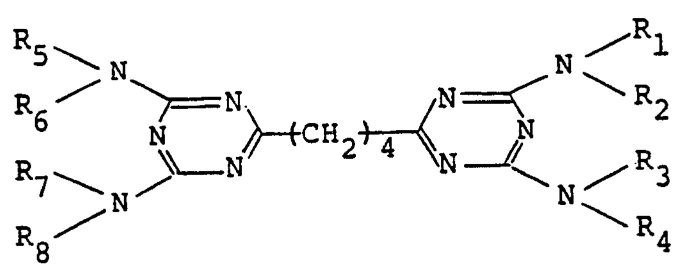

wherein $R_1$, $R_2$, $R_3$, $R_4$, $R_5$, $R_6$, $R_7$ and $R_8$ are independently -H, $-CH_2OH$ or $-CH_2OCH_3$, with the proviso that not all of $R_1$ through $R_8$ are simultaneously hydrogen.

EP 0 391 665 A1

# AMINO COMPOUNDS, AMINO RESINS OBTAINED THEREFROM, PRODUCTION OF AMINO RESINS AND RESIN COMPOSITIONS FOR WATER-BORNE COATINGS

## BACKGROUND OF THE INVENTION

This invention relates to amino compounds, amino resins, process for preparing said resins, and resin compositions for water-borne coatings.

Coatings are used in a wide range of commercial products such as cars, industrial machines, steel-made furniture, electric appliances, can containing foods or drinks, etc. Coatings based on organic solvents have been popularly used since long, but the increasing necessity of saving of resources and energy and also regulations on use of solvents for preventing atmospheric pollution in recent years have urged development of water-borne coatings in place of conventional organic solvent type coatings. Hitherto, as amino resins applied to water-borne coatings, there have been used melamine resins prepared by methyletherifying methylolmelamine.

The coatings comprising melamine resins, however, have defects in that they are poor in boiling water resistance and flexibility.

## SUMMARY OF THE INVENTION

The present invention is intended to provide novel amino compounds, novel amino resins, process for producing said resins, and resin compositions for water-borne coatings having excellent boiling water resistance and flexibility.

In accordance with this invention, there is provided an amino compound represented by the formula:

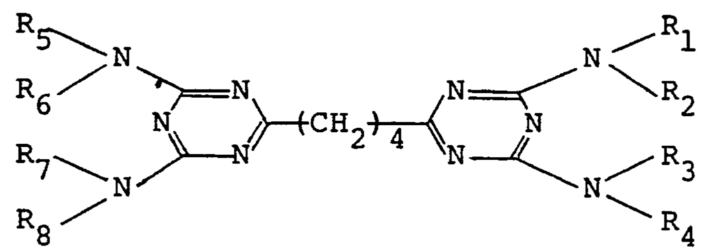

(I)

wherein $R_1$, $R_2$, $R_3$, $R_4$, $R_5$, $R_6$, $R_7$ and $R_8$ represent independently -H, $-CH_2OH$ or $-CH_2OCH_3$, provided that $R_1$ through $R_8$ can not be hydrogen at the same time.

There is also provided according to this invention an amino resin comprising an amino compound of the formula (I) as a principal component and polymer(s) of said compound as secondary component.

The present invention further provides a process for preparing an amino resin characterized in that tetramethylenediguanamine of the formula:

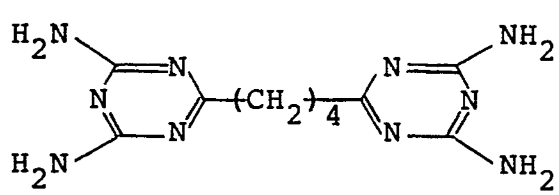

(II)

is converted into a methylolguanamine, which is then methyletherified.

It is also envisaged in this invention to provide a resin composition for water-borne coatings comprising (A) an amino resin obtained by converting tetramethylenediguanamine of the formula:

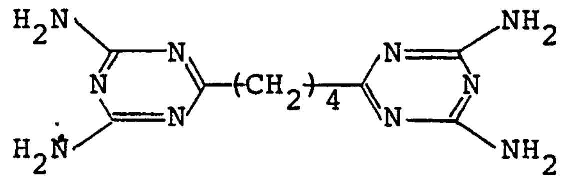 (II)

into methylolguanamine and then methyl-etherifying it, and (B) a resin reactable with said amino resin.

## BRIEF DESCRIPTION OF THE DRAWINGS

FIG. 1 is an IR absorption spectrum of tetramethylenediguanamine obtained in the Synthesis Example.

FIG. 2 is an IR absorption spectrum of amino resin A-I obtained in Example 1.

FIG. 3 is a gel permeation chromatogram of amino resin A-I obtained in Example 1.

FIG. 4 is an IR absorption spectrum of amino resin A-II obtained in Example 2.

FIG. 5 is a gel permeation chromatogram of amino resin A-II obtained in Example 2.

FIG. 6 is an IR absorption spectrum of amino resin A-III obtained in Example 3.

FIG. 7 is a gel permeation chromatogram of amino resin A-III obtained in Example 3.

## DESCRIPTION OF THE PREFERRED EMBODIMENTS

Tetramethylenediguanamine of the formula (II) used in the present invention is a compound already known in the art. It can be synthesized according to the method shown, for instance, in M.J. Booth et al: Chemistry and Industry, p. 1047, 3 Aug., 1968; Japanese Patent Application Kokai (Laid-Open) No. 50-81982, and other publications. The synthesis comprises reacting adiponitrile and dicyandiamide in a solvent diethylene glycol monomethyl ether by using potassium hydroxide as catalyst.

In the present invention, conversion of tetramethylenediguanamine into methylolguanamine can be accomplished, for instance, by reacting tetramethylenediguanamine and formaldehyde at a temperature of 60 to 75 °C for 1 to 8 hours in water and/or alcohol as solvent under an alkaline condition with pH 10.5-12. The alcohols usable as solvent in this reaction include methyl alcohol, ethyl alcohol, butyl alcohol and mixtures thereof. Among them, methyl alcohol (methanol) is preferred as this solvent can serve as a reactant in the succeeding methyl-etherification reaction. The alkaline condition under which the reaction is to be carried out can be brought about by adding sodium hydroxide, potassium hydroxide or the like to the reaction system in an amount sufficient to make the pH of the reaction system 10.5 to 12. The tetramethylenediguanamide/formaldehyde molar ratio in the reaction is approximately in the range of 1/8 to 1/30. Paraformaldehyde may be used in place of formaldehyde.

The average number of methylol groups in the methylolguanamine can be calculated from the amount of formaldehyde consumed in the reaction, which has been determined by quantifying unreacted formaldehyde present in the reaction system according to, for example, the method of JIS K-1502. It is desirable that the average number of methylol groups is not less than 3.5. If said number is less than 3.5, the reaction product may prove rather bad in boiling water resistance.

In the present invention, methyl-etherification can be effectuated by reacting said methylolguanamine with methanol at a temperature of 60 to 75 °C for 1 to 8 hours under an acidic condition with pH 2-4. The reaction is carried out at a methylolguanamine/methanol molar ratio in the range of approximately 1/20 to 1/60. The reaction system can be made acidic by adding, for instance, nitric acid in an amount sufficient to let the reaction system stay at pH 2-4.

The amino resin (A) in accordance with this invention comprises as principal component an amino compound represented by the following formula (I) and contains a polymer or polymers of said amino compound as secondary component:

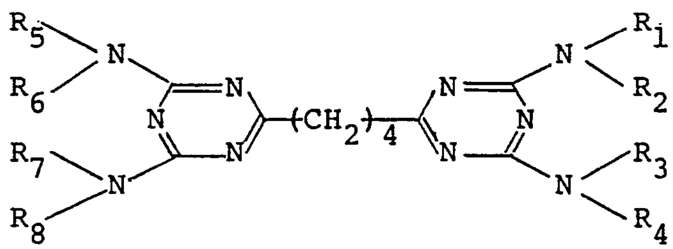

(I)

wherein $R_1$, $R_2$, $R_3$, $R_4$, $R_5$, $R_6$, $R_7$ and $R_8$ represent independently -H, $-CH_2OH$ or $-CH_2OCH_3$, provided that $R_1$ through $R_8$ cannot be hydrogen at the same time.

The amino compound of the formula (I) constituting the main component of the amino resin (A) of this invention is capable of having up to 8 hydrophilic functional groups ($-NCH_2OH$ or $-NCH_2OCH_3$, as represented by $R_1$ through $R_8$) bonded to two triazine rings per molecule and has good water solubility. Also, since this amino compound can contain, per molecule, up to 8 functional groups (-H, $-CH_2OH$ or $-CH_2OCH_3$ as $R_1$ - $R_8$) which are associated with crosslinking, the produced coating film has excellent boiling water resistance. It is to be noted in this connection that the main component of the afore-mentioned melamine resin has 6 functional groups per molecule.

The amino resin (A) of this invention has a structure in which triazine rings are linked by four flexible methylene groups, and this structure contributes to the flexibility of the coatings obtained therefrom.

As resin (B) reactable with said amino resin (A), there can be used known hydroxyl group-containing polyester resins, alkyd resins, acrylic resin, acryl-modified polyester resins and the like. It is preferred to use an alkyd resin obtainable by reacting a polyvalent carboxylic acid, a polyhydric alcohol and, if necessary, an oil or fatty acid. Examples of the polyvalent carboxylic acids usable in the above reaction are phthalic acid, isophthalic acid, terephthalic acid, tetrahydrophthalic acid, maleic acid, fumaric acid, succinic acid, adipic acid, sebacic acid, trimellitic acid, pyromellitic acid and the like. They may be used in the form of an acid anhydride or an ester-forming derivative thereof such as methyl ester.

Examples of the polyhydric alcohols usable in the above reaction are ethylene glycol, diethylene glycol, triethylene glycol, propylene glycol, dipropylene glycol, neopentyl glycol, 1,4-butanediol, 1,6-hexanediol, trimethylene glycol, glycerin, trimethylolpropane, trimethylolethane, pentaerythritol and the like. The oils usable in the above reaction include tung oil, linseed oil, soybean oil, dehydrated castor oil, safflower oil, castor oil, coconut oil, tall oil and the like.

Known methods can be used for preparing a desired alkyd resin. For instance, in case of using an oil for the reaction, an oil and a polyhydric alcohol are reacted at 200 to 260°C in the presence of an ester exchange catalyst such as lithium hydroxide, and the resulting reaction mixture is added with a polybasic acid and the remaining portion of polyhydric alcohol and further reacted at 180 to 250°C. In case no oil is used, the reactants are mixed and reacted at 180 to 250°C.

It is also recommendable to use an acrylic resin obtainable by copolymerizing an $\alpha,\beta$-monoethylenic unsaturated carboxylic acid such as acrylic acid, methacrylic acid, maleic acid, itaconic acid, etc., an $\alpha,\beta$-ethylenically unsaturated monomer having a hydroxyl group such as 2-hydroxyethyl acrylate, 2-hydroxypropyl acrylate, 2-hydroxyethyl methacrylate, 2-hydroxypropyl methacrylate, etc., and other type of unsaturated monomer. As examples of "other type of unsaturated monomer", there can be cited $\alpha,\beta$-monoethylenic unsaturated carboxylic acid alkyl esters such as methyl acrylate, ethyl acrylate, n-butyl acrylate, 2-ethylhexyl acrylate, methyl methacrylate, n-butyl methacrylate, etc.; acrylamide and derivatives thereof such as methacrylamide, N-methylolacrylamide, N-methylolmethacrylamide, diacetoneacrylamide, etc.; $\alpha,\beta$-monoethylenically unsaturated carboxylic acid glycidyl esters such as glycidyl acrylate, glycidyl methacrylate, etc.; saturated carboxylic acid vinyl esters such as vinyl acetate, vinyl propionate, etc.; and aromatic unsaturated monomers such as styrene, $\alpha$-methylstyrene, vinyltoluene, etc.

Said copolymerization can be accomplished by heating the reactants to 130 to 160°C in the presence of a radical polymerization catalyst such as azobisisobutyronitrile, benzoyl peroxide, dibutyl peroxide, cumene hydroperoxide, etc.

Said alkyd or acrylic resin used as resin (B) in the preparation of resin composition according to this invention is preferably so adjusted that it will have an acid value of 20 to 100 and a hydroxyl value of 15 to 200. A too small acid value (less than 20) of said resin tends to give an adverse effect to water solubility and/or water dispersibility of the resin after neutralization, while a too large acid value (above 100) of said resin tends to cause a degradation of coating film properties. Also, a too small hydroxyl value (below 15) of

said resin tends to lead to poor curing characteristics of the composition and a too large hydroxyl value (above 200) has a likelihood of deteriorating water resistance of the coating film.

An advisable method for making said alkyd or acrylic resin soluble or dispersible in water is to neutralize acid groups of the resin with a volatile base such as ammonia or amines. Preferred examples of amines to be used for the above purpose are primary, secondary and tertiary aliphatic or alicyclic amines such as monopropylamine, monobutylamine, diethylamine, dibutylamine, triethylamine, tributylamine, monoethanolamine, ethylmonoethanolamine, monocyclohexylamine, formalin and piperidine. The amount of ammonia or amine to be used for effecting said neutralization is preferably 0.3 to 1.2 moles to one equivalent of acid group.

The amino resin (A)/resin (B) ratio (by weight) is variable depending on the type of resins combined, purpose of use and other factors, but it is usually preferred that said ratio is in the range of 5/95 to 50/50.

The resin composition for water-borne coatings according to this invention may be diluted with a solvent when applied to practical use. Water is favored for use as the solvent, but it is also possible to use other organic solvents soluble in water such as alcohols, ethylene glycol monoethers, etc., in combination with water. More specific examples of such organic solvents are isopropanol, n-butyl alcohol, t-butyl alcohol, methyl Cellosolve, ethyl Cellosolve, butyl Cellosolve and the like. In the diluted resin composition for water-borne coatings, the ratio (by weight) of water/solvent other than water is preferably in the range of 5/1 to 15/1.

In the present invention, known acid catalysts such as dinonylnaphthalenedisulfonic acid, p-toluenesulfonic acid, phthalic anhydride, etc., can be used as a cure accelerator. The amount of such an acid catalyst used for said purpose is preferably less than 1% by weight based on the amino resin (A).

The resin composition for water-brone coatings according to this invention may be added with a pigment and other additives in accordance with the object of application.

The present invention will hereinafter be described more particularly with reference to the examples thereof.

Synthesis Example 1

Synthesis of tetramethylenediguanamine

Tetramethylenediguanamine was synthesized according to the method described in M.J. Booth et al: Chemistry and Industry, p. 1047, 3rd August, 1968. That is, 1 mole of adiponitrile and 2.5 moles of dicyandiamide were reacted in a dimethyl sulfoxide solution in the presence of 1 mole of sodium methylate at 140°C for 2 hours. The yield was 99.5%, and the melting point of the product was 294 - 295°C. An IR absorption spectrum of tetramethylenediguanamine thus obtained is shown in FIG. 1.

It is seen from FIG. 1 that there occurred N-H stretching vibration [*1] at 2,800 - 3,700 $cm^{-1}$, N-H deformation vibration [*2] at 1,680 $cm^{-1}$, triazine ring in-plane deformation vibration [*3] at 1,570 and 1,490 $cm^{-1}$, and triazine ring out-of-plane deformation vibration [*4] at 830 $cm^{-1}$.

The analyses were performed under the conditions described below.

IR absorption spectral analysis

Conducted by using an IR spectrophotometer Model 260-30 manufactured by Hitachi, Ltd.

Gel permeation chromatographic analysis

Column: Gelpack R-420, R-430 and R-440 (mfd. by Hitachi Chemical Co., Ltd.) connected in series

Column size: 10.7 mm in inner diameter and 30 cm in length

Carrier: tetrahydrofuran

Flow rate: 1.7 ml/min

Detector: differential refractometer

The above analytical conditions were applied to the determinations of the synthesis products in the following Examples.

Example 1

Synthesis of amino resin A-I

75.0 g (2 mol calculated as formaldehyde) of paraformaldehyde (purity: 80%, mfd. by Mitsui Toatsu Chemicals Inc.) and 53.3 g (1.7 mol) of methanol were supplied into a 300-cm$^3$ flask equipped with a condenser, a stirrer and a thermocouple for temperature control, and the mixture was adjusted to pH 11.3 with a 30% by weight aqueous solution of sodium hydroxide. The mixed solution was heated to 50°C under stirring. When a homogeneous solution was formed, it was added with 18.4 g (0.07 mol) of tetramethylenediguanamine obtained in the manner described above and the mixture was heated to 60°C and reacted for 5 hours to obtain an oxymethylated substance of tetramethylenediguanamine (hereinafter referred to as "methylolguanamine"). The average number of methylol groups in this methylolguanamine was 6.0. The average number of methylol groups was calculated based on the amount of formaldehyde consumed in the reaction, which has been determined by quantifying unreacted formaldehyde present in the reaction system according to the method of JIS K-1502.

Then the above reaction solution was added with 53.3 g of methanol, adjusted to pH 3.5 with nitric acid, heated to 60°C and reacted for 3 hours. After cooling the reaction solution was adjusted to pH 9.5 or above with a 30% by weight aqueous solution of sodium hydroxide, desolvated by vacuum distillation and filtered. To the resulting solution was added butyl Cellosolve to obtain a yellowish transparent resin solution with a non-volatile content of about 75% by weight (measured at 108°C for 3 hours). Gardner viscosity of this resin solution at 25°C was H - I and the color number according to Gardner color scale was less than 1. Samples of this resin solution were also subjected to IR absorption spectral analysis and gel permeation chromatographic analysis. The results of these analyses are shown in FIG. 2 and FIG. 3, respectively. In order to avoid progress of condensation reaction of resin, all the analyses were conducted by using the samples in the state of a butyl Cellosolve solution.

It is observed that in FIG. 2, N-H stretching vibration (at 2,800 - 3,700 cm$^{-1}$) and N-H deformation vibration (at 1,680 cm$^{-1}$) seen in FIG. 1 disappeared. It is also noted that there occurred C-O-C stretching vibration (*4) of etherified methylol groups (methoxymethylene groups) at 1,090 cm$^{-1}$ and CO stretching vibration (*5) of methylol groups at 1,010 cm$^{-1}$. (*1: OH stretching vibration; *2: aliphatic OH stretching vibration; *3: triazine ring in-plane deformation vibration (at 1,550 and 1,490 cm$^{-1}$); *6: triazine ring out-of-plane deformation vibration (at 810 cm$^{-1}$)). Qauntification by calculation of peak areas of the gel permeation chromatogram of FIG. 3 revealed 86% by weight of monomer, 11% by weight of dimer and 3% by weight of trimer.

Example 2

Synthesis of amino resin A-II

71.3 g (1.9 mol calculated as formaldehyde) of paraformaldehyde (purity: 80%, mfd. by Mitsui Toatsu Chemicals Inc.) were supplied into a 300-cm$^3$ flask provided with a condenser, a stirrer and a thermocouple for temperature control, and the mixture was adjusted to pH 11.3 with a 30% by weight aqueous solution of sodium hydroxide. The mixed solution was heated to 50°C under stirring. When a homogeneous solution was formed, it was added with 27.6 g (0.1 mol) of tetramethylenediquanamine obtained in the manner described above, and the mixture was heated to 60°C and reacted for 5 hours to obtain methylolguanamine. The average number of methylol groups in this methylolguanamine was 5.0.

Then the above reaction solution was added with 60.8 g of methanol, adjusted to pH 3.5 with nitric acid, heated to 60°C and reacted for 3 hours. After cooling, the synthetic solution was adjusted to pH 9.5 or above with a 30% by weight aqueous solution of sodium hydroxide, desolvated by vacuum distillation and filtered. To the resulting solution was added butyl Cellosolve to obtain a yellowish transparent solution with a non-volatile content of about 75% by weight. Gardner viscosity of this resin solution at 25°C was V$^-$ and the color number according to Gardner color scale was less than 1. Samples of this resin solution were also subjected to IR absorption spectral analysis and gel permeation chromatographic analysis. The results of these analyses are shown in FIG. 4 and FIG. 5, respectively.

The assignment in the IR absorption spectrum of FIG. 4 is the same as that in FIG. 2. Quantification by calculation of peak areas of the gel permeation chromatogram of FIG. 5 showed 82% by weight of monomer, 14% by weight of dimer and 4% by weight of trimer.

Example 3

Synthesis of amino resin A-III

60.0 g (1.6 mol calculated as formaldehyde) of paraformaldehyde (purity: 80%, mfd. by Mitsui Toatsu Chemicals Inc.) and 54.4 g (1.7 mol) of methanol were fed into a 300-$cm^3$ flask furnished with a condenser, a stirrer and a thermocouple for temperature control, and the mixture was adjusted to pH 11.3 with a 30% by weight aqueous solution of sodium hydroxide. The mixed solution was heated to 50°C with stirring. When a homogeneous solution was formed it was added with 27.6 (0.1 mol) of tetramethylenediguanamine obtained in the manner described above, and the mixture was heated to 60°C and reacted for 4 hours to obtain methylolguanamine. The average number of methylol groups in this methylolguanamine was 4.0.

Then said reaction solution was added with 54.4 g of methanol, adjusted to pH 3.5 with nitric acid, heated to 60°C and reacted for 3 hours. After cooling, the synthetic solution was adjusted to pH 9.5 or above with a 30 mass% aqueous solution of sodium hydroxide, desolvated by vacuum distillation and filtered. To the resulting solution was added butyl Cellosolve to obtain a yellowish transparent resin solution with a non-volatile content of about 75% by weight. Gardner viscosity of this resin solution at 25°C was $U^-$ and the color number according to Gardner color scale was less than 1. Samples of this resin solution were also subjected to IR absorption spectral analysis and gel permeation chromatographic analysis, the results of which are shown in FIG. 6 and FIG. 7, respectively.

The assignment in the IR absorption spectrum of FIG. 6 is the same as that in FIG. 2. Quantification by calculation of peak areas of the gel permeation chromatogram of FIG. 7 showed 68% by weight of monomer, 19% by weight of dimer, 8% by weight of trimer and 5% by weight of tetramer.

Examples 4 - 6 and Comparative Example 1

Amino resin A-I obtained in Example 1, amino resin A-II obtained in Example 2, amino resin A-III obtained in Example 3 and a melamine resin were blended respectively with a water-soluble acrylic resin (Hitaloyd 7200K, mfd. by Hitachi Chemical Co., Ltd., solid content: 50% by weight, solvent: water/isopropanol) having a hydroxyl value of 22 and an acid value of 33, used as resin (B) reactable with amino resin (A), at the ratios shown in Table 1 to prepare the corresponding resin compositions for water-borne coatings. Each of the thus obtained compositions was coated on a tinplate by using a bar coater #18 so that the dry coating thickness would become 7 µm, and then cured at 170°C for 10 minutes to form a test piece.

Each test piece was subjected to evaluations relating to pencil hardness, Erichsen value, impact value and boiling water resistance. The results are shown in Table 2.

The testing methods used for the evaluations are as follows:

(1) Pencil hardness

Judged by using pencils ("Uni", a trade name mfd. by Mitsubishi Pencil Co., Ltd.)

(2) Erichsen value

Determined according to the testing method of JIS K-5400.

(3) Impact value

Determined by Du Pont impact tester (1/2″, 500 g)

(4) Boiling water resistance

Each test piece was immersed in boiling water for one hour and then the condition of the coating film surface was judged visually.

O : No change.

Δ : Slightly attacked.

x : Heavily attacked.

Table 1:

| Coating formulation | | | | |
|---|---|---|---|---|
| · | Example 4 | Example 5 | Example 6 | Comp. Example 1 |
| Amino resin A-I | 16 | - | - | - |
| Amino resin A-II | - | 16 | - | - |
| Amino resin A-III | - | - | 16 | - |
| Melamine resin* | - | - | - | 13 |
| Hitaloyd 7200K | 56 | 56 | 56 | 56 |
| Water | 28 | 28 | 28 | 31 |

* Melan 523 mfd. by Hitachi Chemical Co., Ltd.
(Note) Dinonylnaphthalenesulfonic acid was added in each coating composition as cure accelerator in an amount of 0.2% by weight based on non-volatile content.

Table 2:

| Coating film quality | | | | |
|---|---|---|---|---|
| | Example 4 | Example 5 | Example 6 | Comp. Example 1 |
| Pencil hardness | 3H | 3H | 3H | 2H |
| Erichsen value (mm) | >6 | >6 | >6 | 3.2 |
| Impact value (cm) | 45 | 45 | 40 | 25 |
| Boiling water resistance | O | O | Δ | × |

Comparative Example 2

Synthesis of butyletherified tetramethylenediguanamine resin

75.0 g (2 mol calculated as formaldehyde) of paraformaldehyde (mfd. by Mitsui Toatsu Chemicals Inc.; purity: 80%) and 64.0 g (0.9 mol) of n-butanol were supplied into a 300-$cm^3$ flask equipped with a condenser, a stirrer and a thermocouple for temperature control, and the mixture was adjusted to pH 11.3 with a 30 mass% aqueous solution of sodium hydroxide. The mixed solution was heated to 50° C under stirring. When a homogeneous solution was formed, it was added with 18.4 g (0.07 mol) of tetramethylenediguanamine, heated to 60° C and reacted for 5 hours to obtain methylolguanamine. The average number of methylol groups in this methylolguanamine was 5.7. The vaerage number of methylol groups was calculated from the amount of formaldehyde consumed by the reaction, which has been determined by quantifying unreacted formaldehyde present in the reaction system according to the method of JIS K-1502.

Then the above reaction solution was added with 64.0 g of n-butanol, adjusted to pH 3.5 with nitric acid, heated to 60° C and reacted for 3 hours. After cooling, the reaction solution was adjusted to pH 9.5 or above with a 30 wt% aqueous solution of sodium hydroxide, desolvated by vacuum distillation and filtered. To the resulting solution was added butyl Cellosolve to obtain a yellowish transparent resin solution with a

non-volatile content of about 75% by weight (measuring conditions: 108° C and 3 hours).

The resin obtained here and the novel amino resin A-I obtained in Example 1 were subjected to evaluation of their dilutability with water. The results are shown in Table 3. Dilutability with water was evaluated by the amount of water at the point when transmittance measured at 600 nm by using an absorptiometer has reacted 50% after starting bit-by-bit dilution of 100 g of each resin solution (butyl Cellosolve solution of each resin, non-volatile content: 75% by weight) with water. It is seen from Table 3 that the amino resin A-I of Example 1 is far superior to the resin of Comparative Example 2 in dilutability with water.

It was tried to prepare a composition for water-borne coatings by following the same procedure as Example 4 except that the resin solution obtained in Comparative Example 2 was used in place of the novel amino resin A-I of this invention, but the obtained composition became cloudy and was unusable as an water-borne coating.

Table 3:

| Result of evaluation of dilutability with water | | |
|---|---|---|
| | Example 1 | Comp. Example 2 |
| Dilutability with water (g of water; determined by using 100 g of resin solution) | 7 | 0.8 |

The resin compositions for water-borne coatings prepared by using novel amino resins mainly composed of novel amino compounds obtained according to this invention have high boiling water resistance and excellent flexibility.

## Claims

1. An amino compound of formula (I):

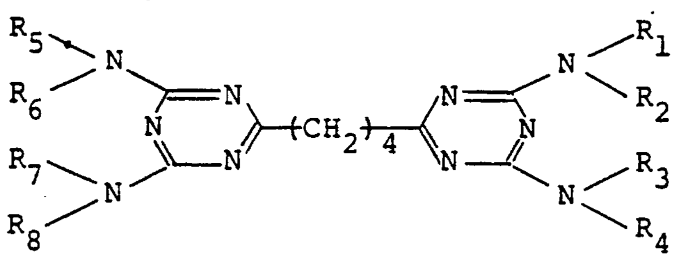

wherein $R_1$, $R_2$, $R_3$, $R_4$, $R_5$, $R_6$, $R_7$ and $R_8$ are independently -H, $-CH_2OH$ or $-CH_2OCH_3$, with the proviso that not all of $R_1$ through $R_8$ are simultaneously hydrogen.

2. A mixture of compounds of formula (I) as defined in claim 1 wherein the average number of $-CH_2OH$ and $-CH_2OCH_3$ groups per molecule is not less than 3.5.

3. An amino resin comprising at least one compound of formula (I) as defined in claim 1 or a mixture of compounds of formula (I) as defined in claim 2 and at least one polymer of a compound of formula (I).

4. A process for producing an amino resin which comprises methyl-etherifying on oxymethylated product of tetramethylenediguanamine of formula (II):

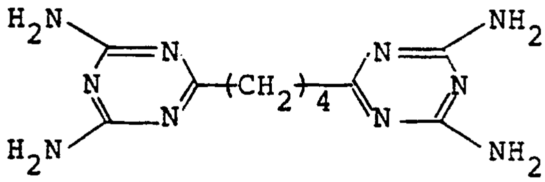

5. A resin composition which comprises (A) an amino resin as defined in claim 3 and (B) a resin reactable with said amino resin.

6. A composition according to claim 5 wherein the weight ratio (A):(B) is from 5:95 to 50:50.

7. A composition according to claim 5 or 6 wherein (B) is a hydroxyl group-containing polyester resin, alkyd resin, acrylic resin or acryl-modified polyester resin.

8. A composition according to any one of claims 5 to 7 which comprises an aqueous solvent.

9. A polymer of at least one compound of formula (I) as defined in claim 1 or of a mixture of compounds of formula (I) as defined in claim 2.

10. A process for coating an article which comprises coating the article with a composition as defined in any one of claims 5 to 8 and curing the composition.

FIG. I

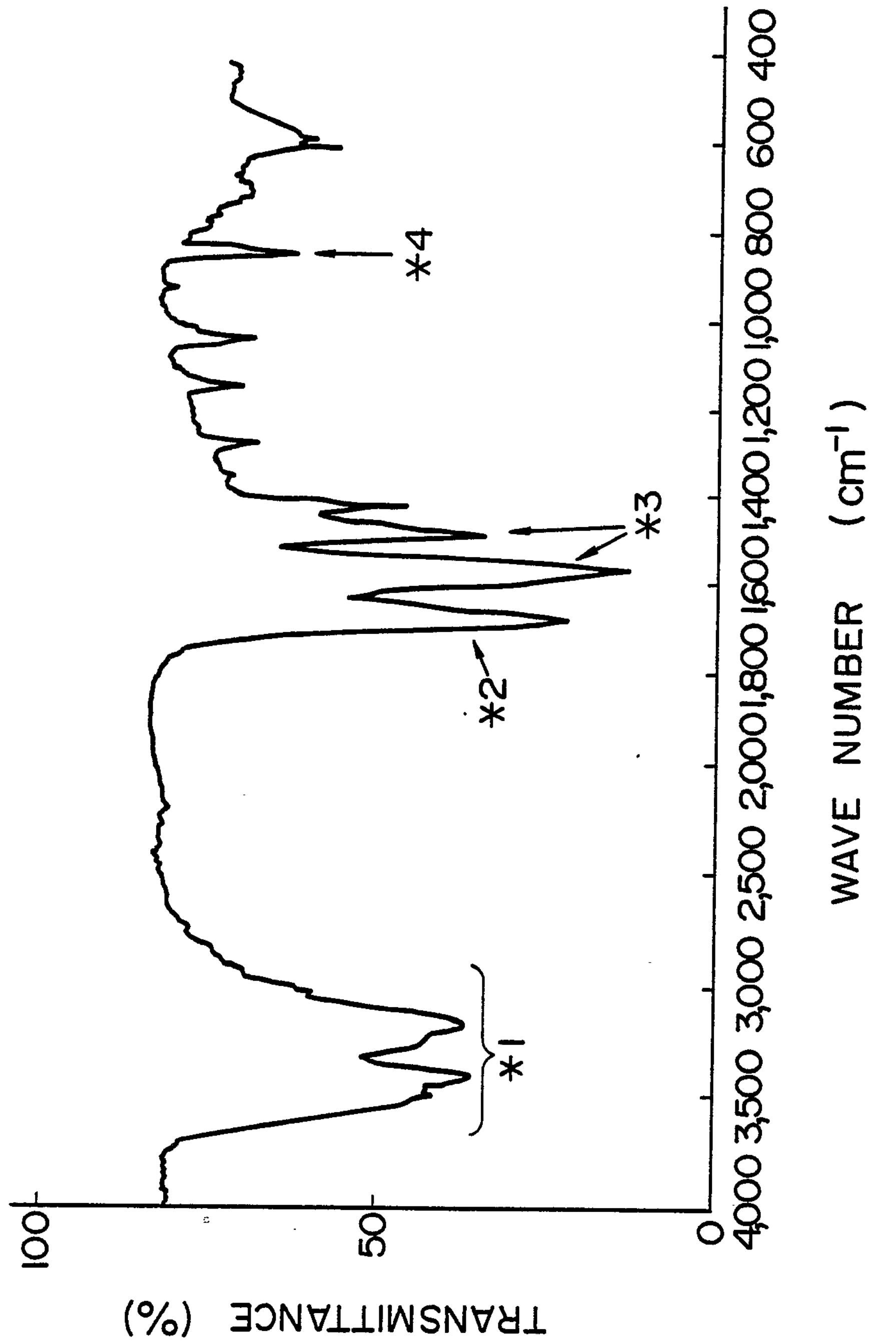

TRANSMITTANCE (%)
100
50
0
4,000
3,500
3,000
2,500
2,000
1,800
1,600
1,400
1,200
1,000
800
600
400
WAVE NUMBER (cm⁻¹)
*1
*2
*3
*4

FIG. 2

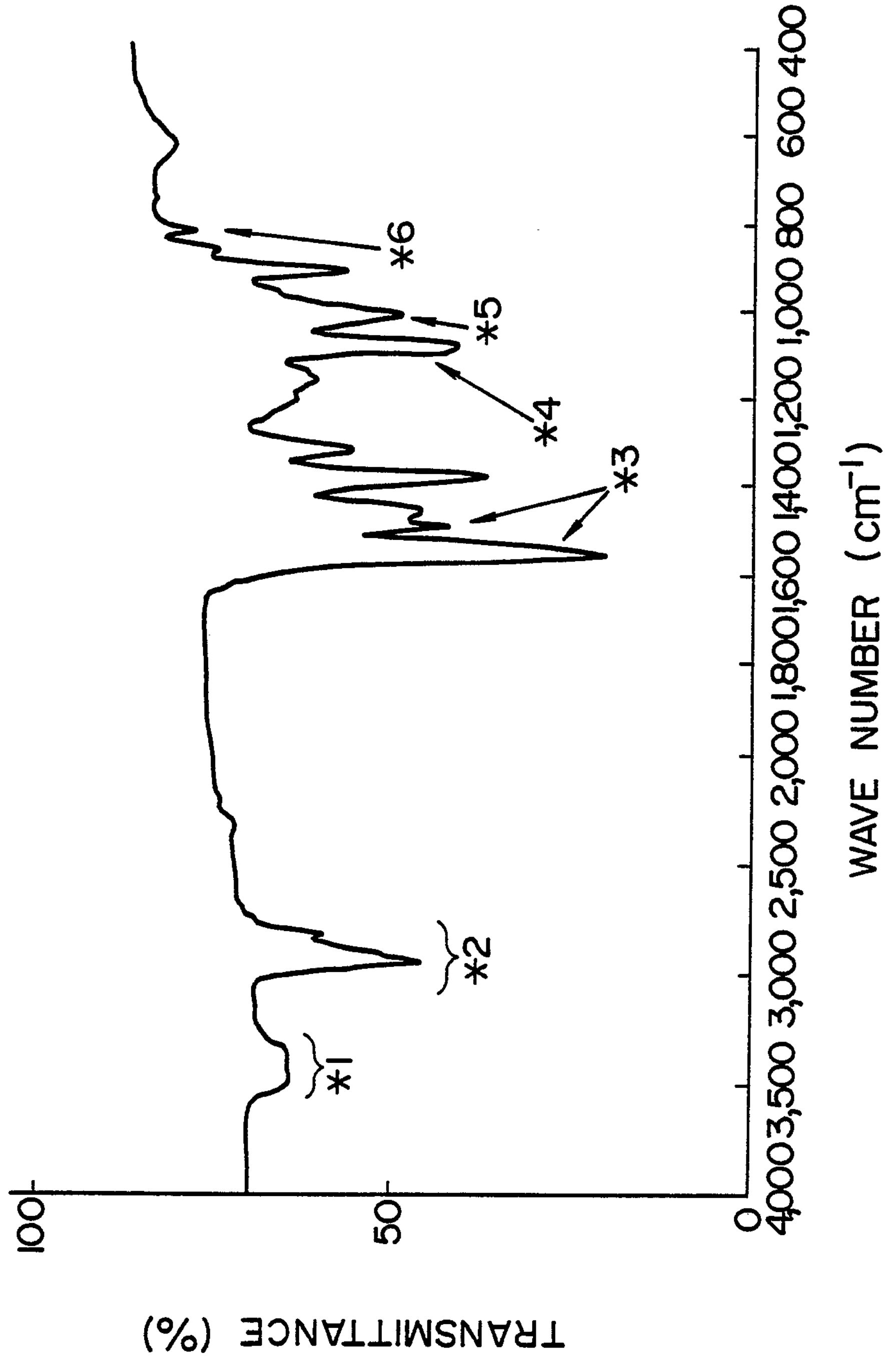
TRANSMITTANCE (%)
100
50
0
WAVE NUMBER (cm⁻¹)
4000 3,500 3,000 2,500 2,000 1,800 1,600 1,400 1,200 1,000 800 600 400
*1
*2
*3
*4
*5
*6

F I G. 3

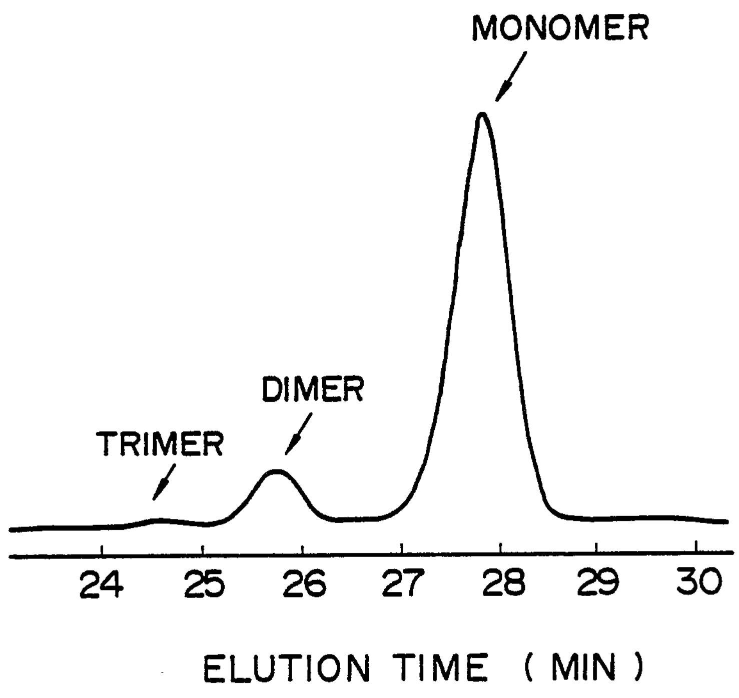

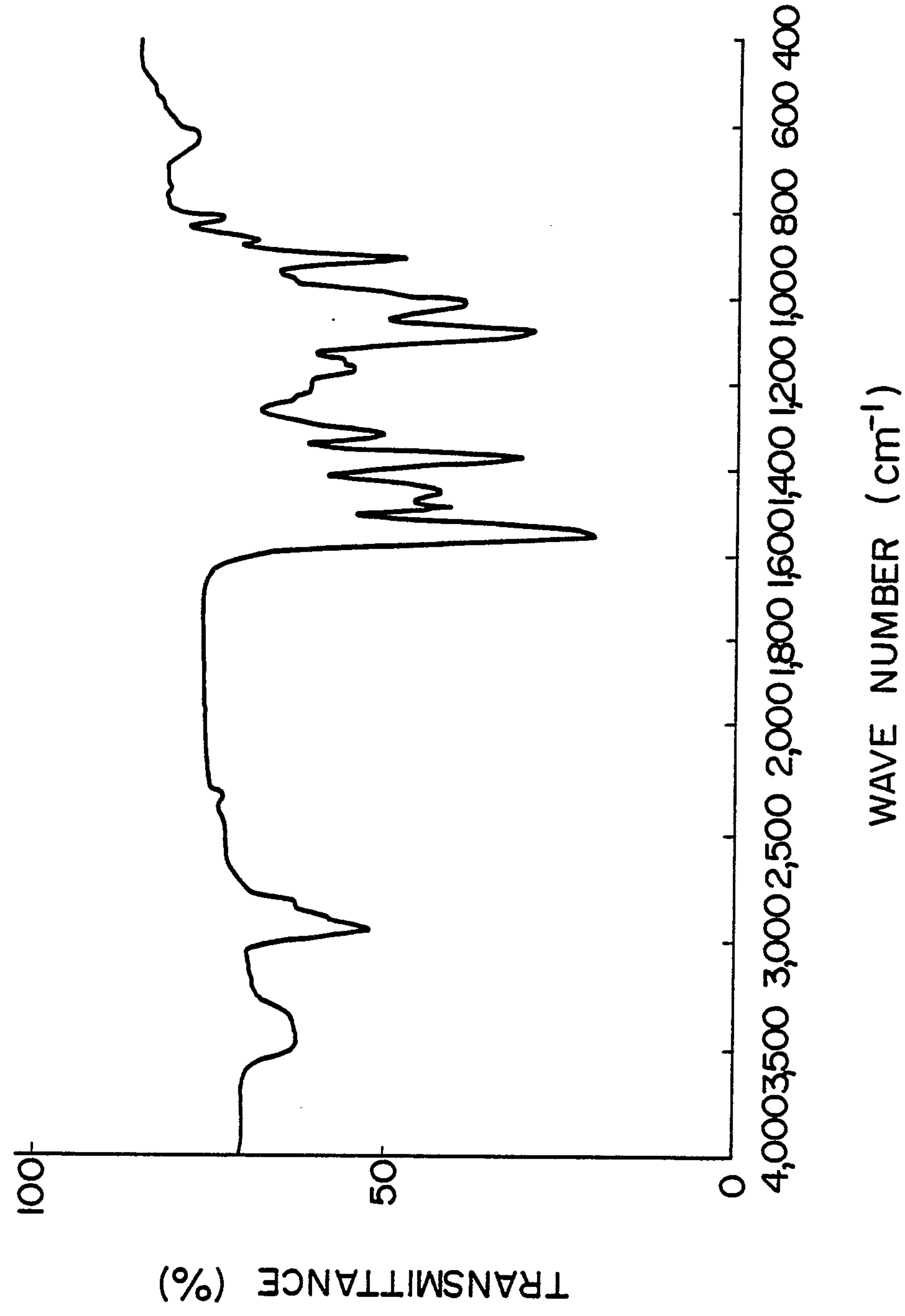
100
50
0
TRANSMITTANCE (%)
4,000
3,500
3,000
2,500
2,000
1,800
1,600
1,400
1,200
1,000
800
600
400
WAVE NUMBER (cm⁻¹)

FIG. 4

FIG. 5

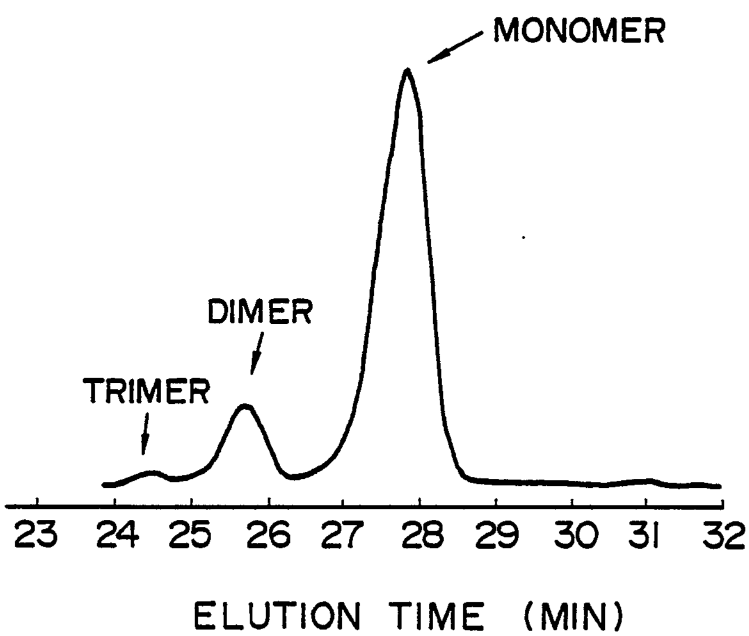
MONOMER
DIMER
TRIMER
23
24
25
26
27
28
29
30
31
32
ELUTION TIME (MIN)

FIG. 6

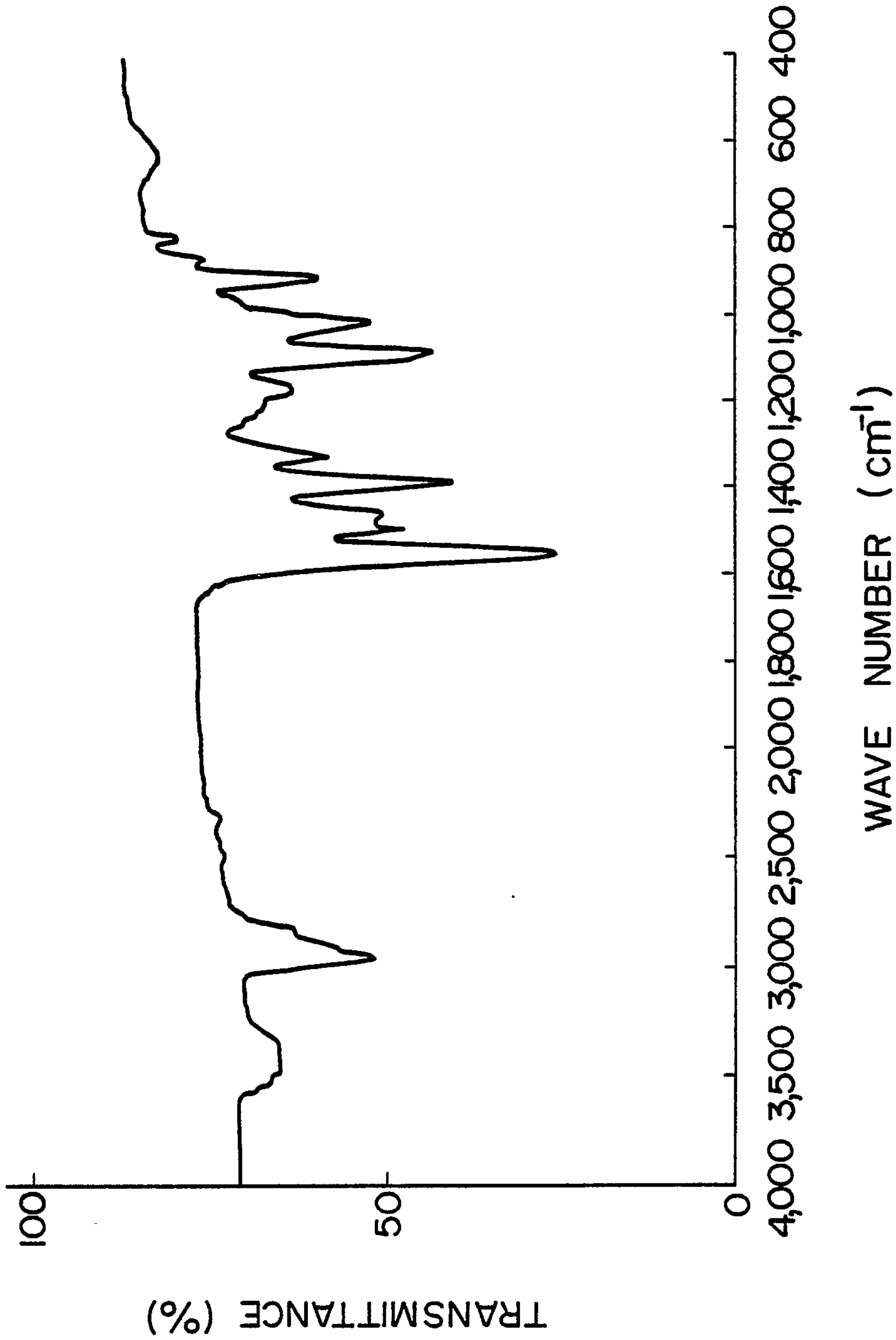
TRANSMITTANCE (%)
100
50
0
4,000
3,500
3,000
2,500
2,000
1,800
1,600
1,400
1,200
1,000
800
600
400
WAVE NUMBER (cm⁻¹)

F I G. 7

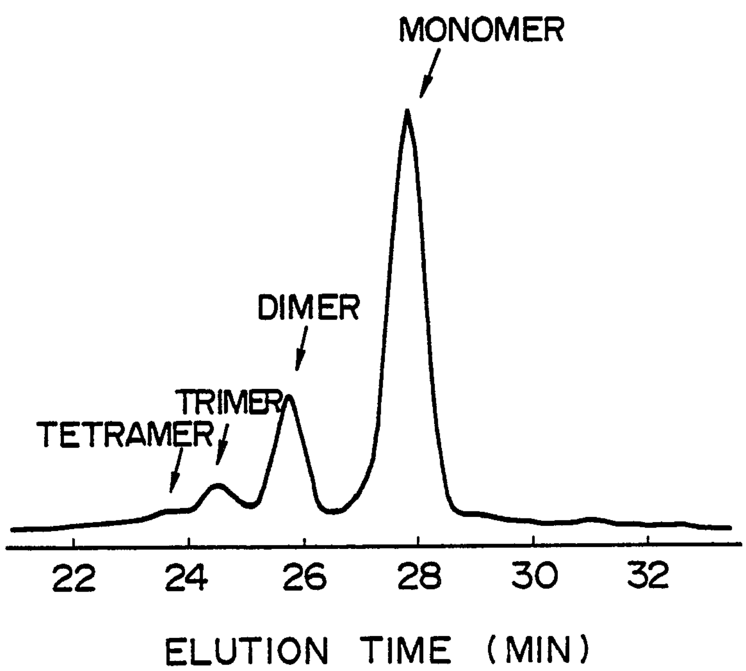
MONOMER
DIMER
TRIMER
TETRAMER
22
24
26
28
30
32
ELUTION TIME (MIN)

European Patent Office

# EUROPEAN SEARCH REPORT

Application number

EP 90303560.8

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| X | CA - A - 1 150 889 (CIBA GEIGY) * Claims 1,9,10 * -- | 1,5 | C 07 D 251/48<br>C 08 G 18/16<br>C 08 G 18/20<br>C 08 G 18/02 |
| A | EP - A1 - 0 044 423 (BAYER) * Example 12 * -- | 1 | |
| A | DD - A3 - 219 031 (TECHNISCHE UNI DRESDEN) * Example 4 * -- | 1,5 | |
| A | CHEMICAL ABSTRACTS, vol. 69, no. 23, December 2, 1968 Columbus, Ohio, USA BOOTH, M.J. et al. "Preparation of diguanamines in dimethyl sulfoxide" page 9053, column 2, abstract-no. 96 674w & Chem. Ind. (London) 1968, (31), 1047 (Eng), J (n=2,3,4 Or 8) are prend in high vields by using -- | 1 | TECHNICAL FIELDS SEARCHED (Int Cl 5)<br>C 07 D 251/00 |
| A | CHEMICAL ABSTRACTS, vol. 78, no. 12, March 26, 1973 Colubus, Ohio, USA PLOTKIN, L.G. et al. "Melamine-formaldehyde resin" page 38, column 2, abstract-no. 73 125q & U.S.S.R. 345,171, 14 Jul 1972 ---- | 1,5 | |

The present search report has been drawn up for all claims

| Place of search | Date of completion of the search | Examiner |
|---|---|---|
| VIENNA | 01-06-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document